# EUROPEAN PATENT SPECIFICATION

(11) **EP 4 192 364 B1**
(45) Date of publication and mention of the grant of the patent: **26.02.2025**
(21) Application number: 20775974.7
(22) Date of filing: 27.08.2020
(51) Int. Cl.: A61B 17/12, A61B 17/00, A61B 90/00

(54) **FLOW REDUCTION CATHETER FOR FLUID OVERLOAD TREATMENT**
FLUSSVERMINDERUNGSKATHETER ZUR BEHANDLUNG VON FLÜSSIGKEITSÜBERLASTUNG
CATHÉTER DE RÉDUCTION D'ÉCOULEMENT DESTINÉ AU TRAITEMENT DE SURCHARGE DE FLUIDE

(43) Date of publication of application: 14.06.2023
(73) Proprietor: Bard Peripheral Vascular, Inc., Franklin Lakes, NJ 07417 (US)
(72) Inventor: CHANDUSZKO, Andrzej, J., Chandler, AZ 85224 (US); CASIRARO, Matt, Tempe, AZ 85284 (US)
(74) Representative: Hoffmann Eitle
(86) International application number: PCT/US2020/048271
(87) International publication number: WO 2022/046060

(56) References cited:
- US-A1- 2005 274 384
- US-A1- 2012 330 348
- US-A1- 2018 280 671

## Description

### BACKGROUND

Congestive heart failure is a condition in which the heart cannot pump enough blood to meet the body's needs. When the heart's blood pumping ability decreases, the kidneys cannot work as they should, which leads to an excess of fluid in the body, termed "Hypervolemia." Hypervolemia symptoms may vary depending on where the fluid is collecting and what other health problems are present. The most common symptoms include unexplained and rapid weight gain, swelling in the arms and legs, abdominal swelling, shortness of breath caused by fluid in the lungs.

There are several approaches to treatment for hypervolemia. One of the most common treatments for hypervolemia is diuretics. Diuretics are drugs that increase the amount of urine the body produces. Increased urination in turn reduces the fluid overload. However, some patients do not respond adequately to diuretic therapy. There is some evidence that by temporarily reducing blood flow to the heart, the symptoms of fluid overload can be improved, leading to increased diuretic efficiency and further improvement to patient's condition.
US 2018/280671 A1 is concerned with a device for treating venous incompetence and related methods. US 2012/330348 A1 is concerned with an embolic implant and method of use.

### SUMMARY

The invention is defined in the independent claim and additional embodiments are defined in the dependent claims. Embodiments disclosed herein are directed to a vessel reshaping device for temporarily limiting blood flow to heart. The vessel reshaping device can provide a simpler construction, have a lower profile, and does not require any membrane to reduce blood flow. Advantageously, the vessel reshaping device mitigates the risk of thrombosis as the low profile frame is less likely to thrombose compared to the membrane, and there is no risk of the membrane detaching or causing thrombosis during delivery, use, or retraction of the device.

The vessel reshaping device generally consists of a frame that can temporarily reshape a vessel, e.g. the inferior vena cava ("IVC") in a single axis extending perpendicular to a direction of flow, thereby reducing the cross-sectional area and blood flow through the vessel. The greater the expansion of the reshaping device, the greater the reduction in cross-sectional area and the reduction in flow. For example, expanding a transverse axis of a substantially circular vessel by 50% can reduce the cross-sectional area of the vessel by substantially 50%. As used herein, the longitudinal axis extends substantially parallel to a direction of flow through the vessel. To note, the vessel reshaping device may not significantly stretch the vessel wall, and instead mainly reshapes the vessel wall, however some stretching may occur.

Disclosed herein is a blood flow regulating device for a vessel including, a delivery catheter extending along a longitudinal axis, and a vessel shaping device having a retracted configuration in the delivery catheter and an expanded configuration out of the delivery catheter, the vessel shaping device being arranged to expand to the expanded configuration along a transverse axis perpendicular to the longitudinal axis of the delivery catheter to reshape the vessel to a flattened configuration.

In some embodiments, the flattened configuration of the vessel defines an extended transverse diameter, a reduced lateral diameter, and a smaller cross-sectional area than the cross-sectional area of the vessel in a resting configuration. The vessel shaping device is formed from Nitinol. The vessel shaping device comprises a frame including a first arm extending transversely outward from a central longitudinal axis to define a first apex, and a second arm extending transversely outward from a central longitudinal axis in an opposite direction from the first arm to define a second apex, a proximal end of the first arm and a proximal end of the second arm are coupled to a proximal collar, and a distal end of the first arm and a distal end of the second arm are coupled to a distal collar.

In some embodiments, the vessel shaping device further comprises a tubular member coupled to the frame. In some embodiments, the tubular member is fixedly attached to a distal collar and slidably engaged with the proximal collar, the distal collar defining an atraumatic tip. In some embodiments, the tubular member is slidably engaged with a distal collar and fixedly attached to the proximal collar, a distal end of the tubular member defining an atraumatic tip. In some embodiments, movement of the tubular member or an actuator rod in one of a proximal direction or a distal direction further expands the vessel shaping device along the transverse axis. The vessel shaping device further comprises a first stability member extending across the first apex from a proximal portion of the first arm to a distal portion of the first arm, and a second stability member extending across the second apex from a proximal portion of the second arm to a distal portion of the second arm. The first stability member or the second stability member extends transversely inward towards the central longitudinal axis.

Also disclosed is a vessel shaping device including, a tubular member extending along a longitudinal axis, and a frame coupled to a distal end of the tubular member, the frame including, a first hinged arm including a first proximal member hingedly coupled to a first distal member to define a first apex, the first proximal member coupled to a proximal collar and the first distal member coupled to a distal collar, and a second hinged arm including a second proximal member hingedly coupled to a second distal member to define a second apex, the second proximal member coupled to the proximal collar and the second distal member coupled to the distal collar.

In some embodiments, one of the proximal collar or the distal collar is threadably engaged with the tubular member. The proximal collar is threadably engaged with one of a right-hand thread or a left-hand thread and the distal collar is threadably engaged with one of a left-hand thread or a right-hand thread. Rotation of the tubular member extends the first apex and the second apex away from a longitudinal axis along the transverse axis to transition the vessel shaping device to the expanded configuration. In some embodiments, the vessel shaping device further includes a first apex member extending substantially parallel to the longitudinal axis and hingedly coupled to the first proximal member and the first distal member, and a second apex member extending substantially parallel to the longitudinal axis and hingedly coupled to the second proximal member and the second distal member. In some embodiments, the vessel shaping device further includes a biasing member disposed between the proximal collar and the distal collar and configured to bias the vessel shaping device towards a retracted configuration. In some embodiments, the vessel shaping device further includes a locking mechanism configured to releasably lock a proximal collar or a distal collar relative to the tubular member to inhibit longitudinal movement thereof.

Also disclosed is a method of regulating blood flow in a vessel including, inserting a delivery catheter into the vessel, moving a vessel shaping device out of a distal end of the delivery catheter, and expanding the vessel shaping device from a retracted configuration to an expanded configuration along a transverse axis perpendicular to a longitudinal axis of the delivery catheter to reshape the vessel from a resting configuration to a flattened configuration.

In some embodiments, the method further includes advancing a tubular member distally relative to the deliver catheter to transition the vessel shaping device to the expanded configuration. In some embodiments, the method further includes withdrawing a tubular member proximally relative to the deliver catheter, after moving the vessel shaping device out of a distal end of the delivery catheter, to transition the vessel shaping device to the expanded configuration. In some embodiments, the method further includes rotating a tubular member relative to the deliver catheter to transition the vessel shaping device to the expanded configuration. In some embodiments, the method further includes distally advancing an actuator rod relative to a tubular member to transition the vessel shaping device to the expanded configuration. In some embodiments, the method further includes locking the vessel shaping device in the expanded configuration. In some embodiments, the method further includes selectively detaching the vessel shaping device from the tubular member. In an embodiment, a portion of the vessel shaping device includes a radiopaque marker.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a side view of a vessel reshaping device in a first configuration, in accordance with embodiments disclosed herein.
FIG. 1B shows a cross-sectional view of a vessel and a distal end view of the vessel reshaping device of FIG. 1A, in accordance with embodiments disclosed herein.
FIG. 1C shows a side view of a vessel reshaping device in a second, expanded configuration, in accordance with embodiments disclosed herein.
FIG. 1D shows a cross-sectional view of a vessel and a distal end view of the vessel reshaping device of FIG. 1C, in accordance with embodiments disclosed herein.
FIG. 2A shows a side view of a vessel reshaping device in a first configuration, in accordance with embodiments disclosed herein.
FIG. 2B shows a cross-sectional view of a vessel and a distal end view of the vessel reshaping device of FIG. 2A, in accordance with embodiments disclosed herein.
FIG. 2C shows a side view of a vessel reshaping device in a second, expanded configuration, in accordance with embodiments disclosed herein.
FIG. 2D shows a cross-sectional view of a vessel and a distal end view of the vessel reshaping device of FIG. 2C, in accordance with embodiments disclosed herein.
FIG. 3A shows a side view of a vessel reshaping device in a first configuration, in accordance with embodiments disclosed herein.
FIG. 3B shows a cross-sectional view of a vessel and a distal end view of the vessel reshaping device of FIG. 3A, in accordance with embodiments disclosed herein.
FIG. 3C shows a side view of a vessel reshaping device in a second, expanded configuration, in accordance with embodiments disclosed herein.
FIG. 3D shows a cross-sectional view of a vessel and a distal end view of the vessel reshaping device of FIG. 3C, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

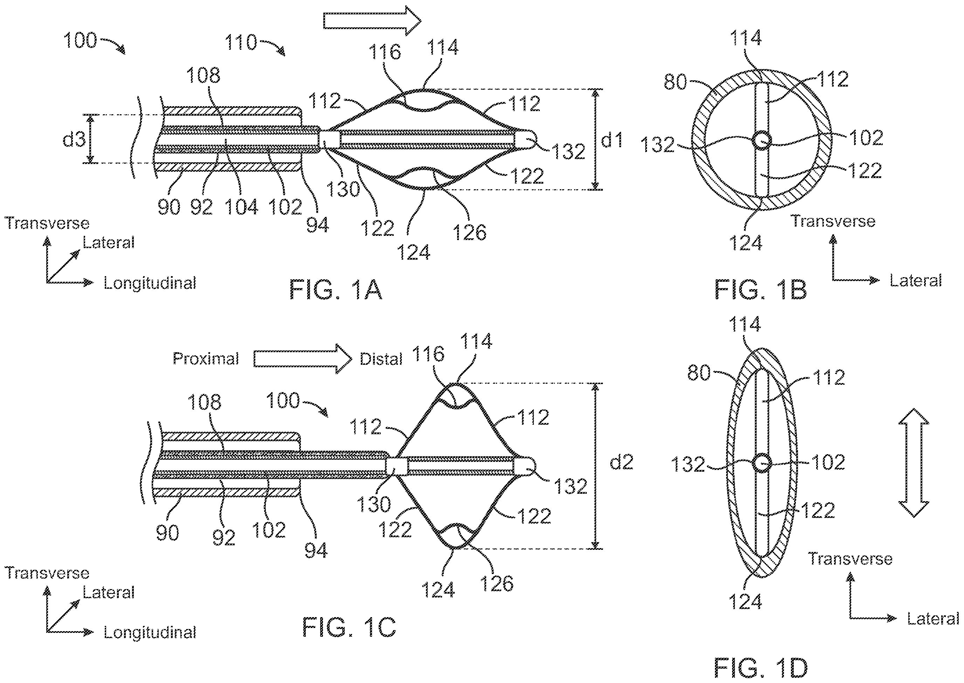

As used herein, and as shown in FIG. 1A, a longitudinal axis extends along an axial length of a catheter, substantially parallel to a direction of flow through a vessel, a lateral axis extends normal to the longitudinal axis, and a transverse axis extends normal to both the longitudinal and the lateral axes.

Unless defined otherwise, all technical and scientific terms used herein have the same meaning as commonly understood by those of ordinary skill in the art.

FIGS. 1A-1D show an embodiment of a vessel shaping device 100 in accordance with embodiments disclosed herein. The vessel shaping device 100 can be disposed at a distal end of a tubular member 102, e.g. stylet, hypotube, or similar elongate medical device configured to extend along the longitudinal axis, through a lumen 92 of a delivery catheter ("catheter") 90. The catheter 90 can include an introducer, catheter, or similar device configured to deliver the vessel shaping device 100 to a target location within a vasculature of the patient. In an embodiment, the tubular member 102 can define a lumen 104 configured to receive a guidewire, actuator rod, or similar elongate device therethrough.

The vessel shaping device 100 can include a frame 110 extending from the tubular member 102 along an axis that extends perpendicular to the longitudinal axis, for example the transverse axis, lateral axis, or combinations thereof. For ease of explanation, embodiments disclosed herein are described as expanding along the transverse axis, however, this is not intended to be limiting and embodiments can expand along the lateral axis or at an angle relative to one of the transverse or lateral axes.

The frame 110 can include a first arm 112 extending in a first direction from the tubular member 102 and defining a first apex 114. The frame 110 can include a second arm 122 extending in a second direction, opposite the first direction, from the tubular member 102 and defining a second apex 124.

In an embodiment, one of a proximal end or a distal end of the first arm 112 can be formed integrally with the tubular member 102. In an embodiment, one of the proximal end or the distal end of the first arm 112 can be slidably engaged with the tubular member 102. In an embodiment, one of a proximal end or a distal end of the first arm 112 can be coupled with a collar that can be coupled with the tubular member 102 in either a fixed relationship or can be slidably engaged with the tubular member 102. For example, the proximal end of the first arm 112 can be coupled with a proximal collar 130 and a distal end of the first arm 112 can be coupled to a distal collar 132. In an embodiment, the distal collar 132 can define an atraumatic tip. In an embodiment, a portion of the frame 100 can include a radiopaque or acoustically opaque material to facilitate viewing the frame under imaging, e.g. X-ray, fluoroscopy, ultrasound, MRI, etc. In an embodiment, one of the proximal collar 130, distal collar 132, first arm 112, second arm 122, first apex 114, second apex 124, portion thereof, or combinations thereof, can include a radiopaque or acoustically opaque material to identify an outer perimeter of the frame 100 under imaging and facilitate correct positioning of the frame 100 within the vessel 80.

In an embodiment, one of a proximal end or a distal end of the second arm 122 can be formed integrally with the tubular member 102. In an embodiment, one of the proximal end or the distal end of the second arm 122 can be slidably engaged with the tubular member 102. In an embodiment, one of a proximal end or a distal end of the second arm 122 can be coupled with a collar that can be coupled with the tubular member 102 in either a fixed relationship or can be slidably engaged with the tubular member 102. For example, the proximal end of the second arm 122 can be coupled with the proximal collar 130 and a distal end of the second arm 122 can be coupled to the distal collar 132.

In an embodiment, one of the first arm 112 or the second arm 122 can include a stability member. For example, the first arm 112 can include a first stability member 116 extending across the first apex 114 between a proximal portion and a distal portion of the first arm 112. The first stability member 116 can extend transversely inward, toward a central longitudinal axis. The first stability member 116 can further support the elastic deformation of first arm 112, as described in more detail herein. Similarly, the second arm 122 can include a second stability member 126 extending across the second apex 124 between a proximal portion and a distal portion of the second arm 122. The second stability member 126 can extend transversely inward, toward a central longitudinal axis. The second stability member 126 can further support the elastic deformation of second arm 122, as described in more detail herein.

In an embodiment, the first arm 112 and the second arm 122 of the frame 110 can elastically deform between a retracted configuration (e.g. FIG. 1A) and an extended configuration (e.g. FIG. 1C).

In an embodiment, the vessel shaping device 100 can be biased towards the retracted configuration, such that at rest the vessel shaping device 100 defines a first transverse distance (*d1*) between the first apex 114 and the second apex 124. In an embodiment, the first transverse distance (*d1*) can be greater than a diameter (*d3*) of the catheter lumen 92. In an embodiment, the first transverse distance (*d1*) can be less than a diameter (*d3*) of the catheter lumen 92. In an embodiment, the vessel shaping device 100 can be biased towards the extended configuration, such that at rest the vessel shaping device 100 defines a second distance (*d2*) between the first apex 114 and the second apex 124, which is greater than the first distance (*d1*). In an embodiment, the vessel shaping device 100 in the retracted configuration defines a transverse distance (*d1*) that is greater than a diameter (*d3*) of the catheter lumen 92. The vessel shaping device 100 can elastically deform to a compact configuration (not shown) where a transverse distance between the first apex 114 and the second apex 124 is less than a diameter (*d3*) of the catheter lumen 92.

As shown in FIG. 1B, in the retracted configuration, the distance (*d1*) between the first apex 114 and the second apex 124 can be equal to, or less than, a diameter of the vessel 80 in a resting position. To note, the vessel 80 in the resting position can define a circular or slightly oval cross-sectional shape. As shown in FIG. 1D, in the extended configuration, the distance (*d2*) between the first apex 114 and the second apex 124 can be greater than a resting diameter of the vessel 80 at a target location. As such, the diameter of the vessel 80 can be reshaped along a first axis, e.g. a transverse axis, to a flattened configuration. As such, in the flattened configuration a second axis of the vessel 80, e.g. a lateral axis, is reduced. Further, a cross-section area of the vessel 80 in the flattened configuration, e.g. FIG. 1D, can be less than a cross-section area of the vessel 80 in a resting configuration, e.g. FIG. 1B. For example, a substantially circular vessel 80 in a resting configuration can be reshaped to a flattened configuration by extending the transverse axis by 50%. In such a flattened configuration, a cross-sectional area of the vessel can be reduced by 50%. However, it will be appreciated that greater or lesser ratios of reshaping to cross-sectional area reduction are also contemplated.

In an exemplary method of use, a vessel shaping device 100 can be provided as described herein. The vessel shaping device 100 can be disposed within a lumen 92 of the delivery catheter 90 and advanced to a target location within the vasculature of the patient. Once proximate the target location, the tubular member 102 can be urged distally to advance the vessel shaping device 100 distally of the distal tip 94 of the catheter 90. The vessel shaping device 100 can then transition to the extended configuration to define a transverse distance (*d2*) between the first apex 114 and the second apex 124 and reshape the vessel 80 from a resting configuration to a flattened configuration and reduce the cross-sectional area of the vessel 80.

In an embodiment, the vessel shaping device 100 can be biased towards the extended configuration and can be elastically deformed to a retracted configuration to fit within the lumen 92 of the catheter 90. As such, when the vessel shaping device 100 is advanced distally of the distal tip 94 of the catheter 90, the device 100 can expand to an expanded configuration and transition the vessel to the flattened configuration.

In an embodiment, the vessel shaping device 100 can be biased towards the retracted configuration that defines a transvers distance (*d1*). As such, when the vessel shaping device 100 is advanced distally of the distal tip 94 of the catheter 90, a handle coupled to a proximal end of the tubular member 102 can be actuated to transition the device 100 to the expanded configuration. In an embodiment, the tubular member 102 can include an actuator, operatively coupled to one of the proximal collar 130 or the distal collar 132 to selectively transition the vessel shaping device 100 between the retracted configuration and the extended configuration. For example, an actuator rod can extend through a lumen 104 the tubular member 102 to the distal collar 132. Withdrawing the actuator rod relative to the tubular member 102 can draw the distal collar 132 towards the proximal collar 130 and transition the vessel shaping device 100 to the expanded configuration. As shown in FIGS. 1A and 1C, in an embodiment, the actuator rod 108 can be slidably engaged with an outer surface of the tubular member 102 and can be coupled to the proximal collar 130. Advancing the actuator rod 108 proximally can advance the proximal collar 130 towards the distal collar and transition the vessel shaping device 100 to the expanded configuration.

In an embodiment, the transverse distance (*d1*) can be greater than a lumen diameter (*d3*). As such the device 100 can be elastically deformed to fit within the lumen 92 of the catheter 90. Advancing the device 100 can allow the device 100 to transition to the retracted configuration, defining the transverse distance (*d1*)*.* Withdrawing the tubular member 102 can then engage a proximal end of the frame 110 against a distal tip 94 of the catheter, urging the proximal collar 130 towards the distal collar 132 and transitioning the vessel shaping device 100 to the extended configuration. Advantageously, a user can modify the configuration of the vessel shaping device 100 between the retracted configuration and the extended configuration to modify a cross-sectional area of the vessel 80 and modify an amount of fluid flow therethrough.

In an embodiment, the vessel shaping device 100 can be formed of a resilient, material, elastically deformable material, malleable material, super-elastic, or shape memory material. In an embodiment, the vessel shaping device 100 can include metal, alloy, stainless steel, Nitinol, plastic, polymer, carbon based material, carbon fiber material, composite materials, combinations thereof, or the like. In an embodiment, the vessel shaping device 100 can be shaped from the retracted configuration to the expanded configuration and remain in the expanded configuration until reshaped.

In an embodiment, the vessel shaping device 100 can be selectively detachable from the tubular member 102. The vessel shaping device 100 can then remain at the target location, reshaping the vessel 80 along the transverse axis to the flattened configuration, while the tubular member 102 and catheter 90 can be withdrawn. In an embodiment, the vessel shaping device 100 can be retrieved by extending the tubular member 102, or similar device, to selectively engage the vessel shaping device 100, e.g. grasping a proximal portion thereof. The tubular member 102 and vessel shaping device 100 can then be withdrawn proximally into the lumen 92 of the catheter 90 and removed from the vasculature of the patient. Advantageously, the stability members 116, 126 can provide extra strength or support to the arms 112, 122 at the location of the apices 114, 124. In an embodiment, the stability members 116, 126 can provide additional elastic strength biasing the vessel shaping device 100 towards one of the extended or retracted configurations, as described herein.

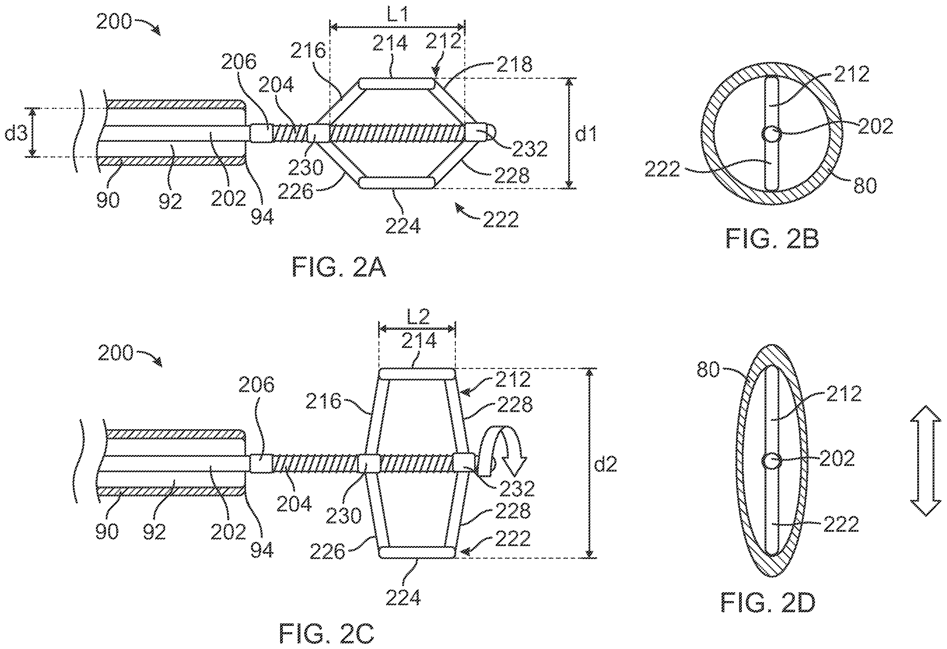

As shown in FIGS. 2A-2D, in an embodiment, a vessel shaping device 200 can include an articulated frame 210 having a first arm 212 and a second arm 222 each extending transversely from the tubular member 202 and each including two or more members hingedly coupled to each other. For example, the first arm 212 can include a proximal member 216 hingedly coupled to a distal member 218 to create an apex 214. In an embodiment, the proximal member 216 can be hingedly coupled to an apex member 214 at a proximal end. The apex member 214 can extend substantially parallel to the tubular member 202 to a distal end that is hingedly coupled to a distal member 218. In an embodiment, a proximal end of the proximal member 216 can be hingedly coupled to a proximal collar 230. A distal end of the distal member 218 can be hingedly coupled to a distal collar 232.

The second arm 222 can include a proximal member 226 hingedly coupled to a distal member 228 to create an apex 224. In an embodiment, the proximal member 226 can be hingedly coupled to an apex member 224 at a proximal end. The apex member 224 can extend substantially parallel to the tubular member 202 to a distal end that is hingedly coupled to a distal member 228. In an embodiment, a proximal end of the proximal member 226 can be hingedly coupled to a proximal collar 230. A distal end of the distal member 228 can be hingedly coupled to a distal collar 232.

In an embodiment, one of the proximal collar 230 or the distal collar 232 can be integrally formed with the tubular member 202. In an embodiment, one of the proximal collar 230 or the distal collar 232 can be threadably engaged with the tubular member 202 and rotating the tubular member 202 can move one of the proximal collar 230 or the distal collar 232 along a longitudinal axis relative to the vessel shaping device 200. In an embodiment, a distal portion of the tubular member 202 can include a threaded portion 204. In an embodiment, the threaded portion 204 can include a multi-lead thread where a single rotation of the tubular member 202 can provide a greater longitudinal movement of one of the proximal collar 230 or the distal collar 232 relative to a single-lead thread. For example, a quad lead thread can provide four times more longitudinal movement per rotation relative to a single lead thread.

In an embodiment, the threaded portion 204 can include a right-handed threaded portion and a left-handed threaded portion. In an embodiment, the proximal collar 230 can be threadably engaged with the right-handed threaded portion and the distal collar 232 can be can be threadably engaged with the left-handed threaded portion. In an embodiment, the proximal collar 230 can be threadably engaged with the left-handed threaded portion and the distal collar 232 can be can be threadably engaged with the right-handed threaded portion. Rotating the tubular member 202 can move both the proximal collar 230 and the distal collar 232 along a longitudinal axis to change a longitudinal distance (*L*) therebetween. As shown in FIG. 2A, a first length (*L1*) between the proximal collar 230 and the distal collar 232 can provide a first transverse distance (*d1*) between the first apex 214 and the second apex 224. Rotating the tubular member 202 can provide a second longitudinal distance (*L2*), which is less than the first longitudinal distance (*L1*)*,* and provides a second transverse distance (*d2*) between the first apex 214 and the second apex 224 that is greater than first distance (*d1*).

In an embodiment, a first transverse distance (*d1*) is equal to or less than a diameter of the vessel 80 in a resting configuration. In an embodiment, a first transverse distance (*d1*) is equal to or less than a diameter (*d3*) of the catheter 90. The second transverse distance (*d2*) is greater than a diameter of the vessel 80 in a resting configuration. As such the vessel reshaping device 200 can reshape the vessel 80 to a flattened configuration by increasing a transverse axis to reduce a lateral axis of the vessel 80 and reduce a cross-sectional area of the vessel 80.

In an embodiment, the reshaping device 200 can be selectively releasable from the tubular member 202. For example, the tubular member 202 can include a release mechanism 206 configured to selectably release a distal portion of the tubular member 202 including the threaded portion 204 and the vessel shaping device 200. As such the vessel shaping device 200 can remain at the target location while the catheter 90 and tubular member 202 are withdrawn.

In an exemplary method of use, a vessel shaping device 200 and threaded tubular member 202 can be provided as described herein. In an embodiment, the longitudinal distance (*L*) between the proximal collar 230 and the distal collar 232 can be configured such that a transverse distance (*d1*) is less than a diameter (*d3*) of the catheter lumen 92. Once at the target location, the tubular member 202 can be urged distally to urge the vessel shaping device 200 distally of the distal tip 94 of the catheter 90. The tubular member 202 can then be rotated to reduce a longitudinal length (*L*) between the proximal collar 230 and the distal collar 232. In turn, this can increase the transverse distance between the first apex 214 and the second apex 224 to distance (*d2*) that is greater than a transverse diameter of the vessel 80 in the resting configuration and reshape the vessel 80 to a flattened configuration (FIG. 2D), as described herein. In an embodiment, the tubular member 202 can selectively release the vessel shaping device 200 by detaching the engagement mechanism 206.

In an embodiment, the vessel shaping device 200 can be removed by reconnecting the engagement mechanism 206 and rotating the tubular member 202 in an opposite direction. The longitudinal distance (*L*) between the proximal collar 230 and the distal collar 232 can be increased which can decrease a transverse distance between the first apex 214 and the second apex 224 from a distance (*d2*) to a distance (*d1*) to allow the vessel 80 to resume the resting configuration. The vessel shaping device 200 can then be withdrawn into the catheter 90 and removed from the vasculature of the patient.

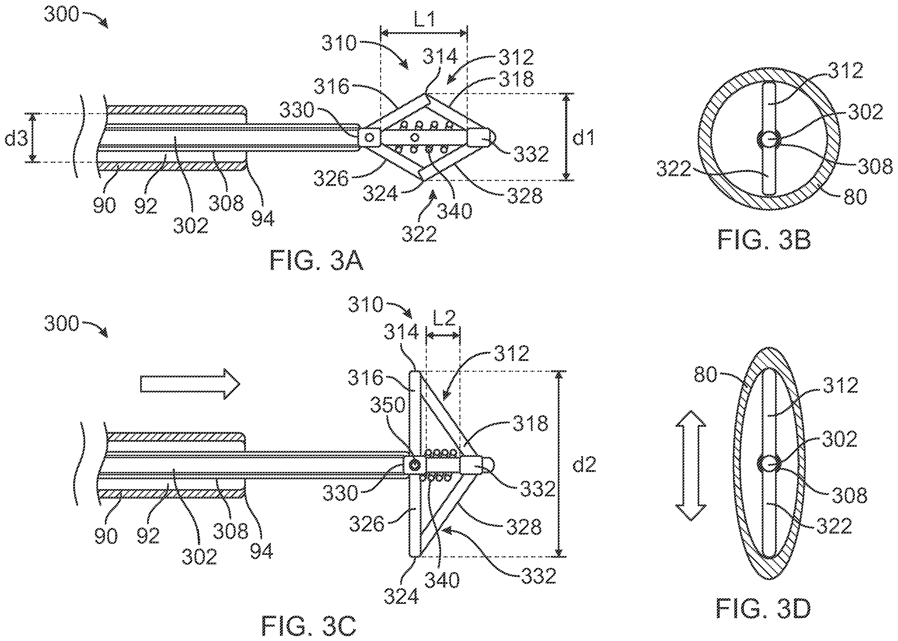

As shown in FIGS. 3A-3D, in an embodiment, a vessel shaping device 300 can include an articulated frame 310 configured to transition between a retracted configuration (FIG. 3A) and an extended configuration (FIG. 3C). The vessel shaping device 300 can include a first arm 312 and a second arm 322 extending transversely from a tubular member 302. The first arm 312 can include a first proximal member 316 hingedly coupled to a first distal member 318 to define a first apex 314. The second arm 322 can include a second proximal member 326 hingedly coupled to a second distal member 328 to define a second apex 324. A proximal end of the proximal member 316 can be hingedly coupled to a proximal collar 330. A distal end of the distal member 316 can be hingedly coupled to a distal collar 332. In an embodiment, the distal collar 332 can define an atraumatic tip. In an embodiment, a distal tip of the tubular member 302 or actuator rod 208 can define an atraumatic tip.

In an embodiment, one of the proximal collar 330 or the distal collar 332 can be fixedly coupled to the tubular member 302. In an embodiment, one of the proximal collar 330 or the distal collar 332 can be slidably engaged with the tubular member 302. In an embodiment, the vessel shaping device 300 can include a biasing member 340 aligned with the longitudinal axis and disposed between the proximal collar 330 and the distal collar 332. The biasing member, e.g. a compression spring, is configured to bias the vessel shaping device 300 towards the retracted configuration.

In the retracted configuration, a transverse distance (*d1*) between the first apex 314 and the second apex 324 can be less than a diameter of the vessel 80 in a resting state. In an embodiment, in the retracted configuration, a transverse distance (*d1*) between the first apex 314 and the second apex 324 can be less than a diameter (*d3*) of the catheter lumen 92.

In an embodiment, the tubular member 302 can include an actuator rod 308. In an embodiment, the actuator rod 308 can be slidably engaged with an interior of the tubular member 302. In an embodiment, the actuator rod 308 can define a sleeve, slidably engaged with an exterior of the tubular member 302. However, it will be appreciated that other configurations of tubular member 302 and actuator rod 308 are also contemplated.

In an embodiment, actuating the actuator rod 308 can slide the proximal collar 330 relative to the distal collar 332 such that a longitudinal distance (*L*) between the proximal collar 330 and the distal collar 332 is reduced which can cause the transverse distance (*d*) between the first apex 314 and the second apex 324 to be increased to transverse distance (*d2*) to extend the transverse diameter of the vessel 80, reducing the lateral diameter of the vessel 80 to transition the vessel 80 to a flattened configuration and reduce the cross-sectional area of the vessel 80, as described herein.

In an embodiment, one of the proximal collar 330 or the distal collar 332 can include a locking mechanism 350. For example, as shown, the proximal collar 330 can including a locking mechanism configured to lock the proximal collar 330 relative to the tubular member 302, and/or actuator rod 308, to secure the vessel shaping device 300 in the extended configuration.

In an embodiment, the locking mechanism 350 can include a dowel slidably engaged with the tubular member 302 or the actuator rod 308 and biased towards a locked position. When the dowel aligns with an aperture disposed on the proximal collar 330, the dowel can transition to the locked position and engage the aperture to inhibit further longitudinal movement of the proximal collar 330. In an embodiment, a second actuator mechanism can be configured to retract the dowel, disengaging the aperture and allow the biasing member 340 to slide the proximal collar 330 relative to the tubular member 302/actuator rod 308 to the retracted configuration. It will be appreciated that various other configurations of locking mechanisms 350 are also contemplated including locking the distal collar 332 to the tubular member 302. Further, various other locking mechanisms 350 are also contemplated including pawls, ratchets, gears, worm-wheels, combinations thereof, or the like.

In an exemplary method of use, a vessel shaping device 300 including a tubular member 302 and actuator rod 308 is provided, as described herein. The vessel shaping device 300 can be disposed within a proximal portion of a catheter 90 and advanced into vasculature of a patient to a target location. The tubular member 302 can be advanced until the vessel shaping device 300 is urged distally of a distal tip 94 of the catheter 90. The actuator rod 308 can be actuated which can compress the biasing member 340 and reduce a longitudinal distance (*L*) between the proximal collar 330 and the distal collar 332. As such, a transverse distance (*d*) between the first apex 314 and the second apex 324 is increased to a transverse distance (*d2*), i.e. the extended configuration, and a cross-sectional shape of the vessel 80 can be reshaped from the resting configuration to a flattened configuration. In an embodiment, in the extended configuration, the locking mechanism 350 can engage to prevent further longitudinal movement of one of the proximal collar 330 or the distal collar 332 to lock the vessel shaping device 300 in the extended configuration.

In an embodiment, the actuator rod 308 is slidably engaged with an interior of the tubular member 302, and can withdraw a distal collar 332 proximally towards the proximal collar 330 to transition the vessel shaping device 300 to the extended configuration. In an embodiment, the actuator rod 308 is slidably engaged with an exterior of the tubular member 302 and can advance a proximal collar 330 distally towards the distal collar 332 to transition the vessel shaping device 300 to the extended configuration. However, it will be appreciated that other combinations of slidable actuator rod 308, proximal collar 330, or distal collar 332 are also contemplated. In an embodiment, the vessel shaping device 300 can be selectively disengaged from the tubular member 302 to remain at the target location while the tubular member 302 and the catheter can be withdrawn.

In an embodiment, a proximal end of the tubular member 302 can include a handle or similar structure configured to facilitate grasping and manipulating the tubular member 302 and vessel shaping device 300 disposed at a distal end thereof. Further the handle can include one or more buttons, levers, or the like configured to actuate the actuator rod 308, locking mechanism 350, or combinations thereof.

In an embodiments, of the vessel shaping device 100, 200, 300, disclosed herein can be formed of a metal, alloy, superelastic alloy, stainless steel, Nitinol, plastic, polymer, carbon based material, carbon fiber, composite material, combinations thereof, or the like. Advantageously, embodiments of the vessel shaping device can be formed of a material that mitigate the formation of thromboses. Further, embodiments of the vessel shaping device provide a low profile structure with a reduced surface area to further mitigate the formation of thromboses. In an embodiment, embodiments of the vessel shaping device, or components thereof, can include a coating, for example an anti-thrombotic coating or the like to further mitigate the formation of thromboses.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the claims appended hereto.

## Claims

1. A blood flow regulating device for a vessel, comprising:
a delivery catheter (90) extending along a longitudinal axis; and
a vessel shaping device (100) having a retracted configuration in the delivery catheter (90) and an expanded configuration out of the delivery catheter (90), the vessel shaping device (100) being arranged to expand to the expanded configuration along a transverse axis, perpendicular to the longitudinal axis of the delivery catheter (90), to reshape the vessel to a flattened configuration, wherein the vessel shaping device (100) comprises a frame (110) including a first arm (112) extending transversely outward from the central longitudinal axis to define a first apex (114), and a second arm (122) extending transversely outward from the central longitudinal axis in an opposite direction from the first arm (112) to define a second apex (124), a proximal end of the first arm (112) and a proximal end of the second arm (122) are coupled to a proximal collar (130), and a distal end of the first arm (112) and a distal end of the second arm (122) are coupled to a distal collar (132), **characterized in that**:
the vessel shaping device (100) further comprises a first stability member (116) extending across the first apex from a proximal portion of the first arm (112) to a distal portion of the first arm (112), and a second stability member (126) extending across the second apex from a proximal portion of the second arm (122) to a distal portion of the second arm (122).

2. The blood flow regulating device according to claim 1, wherein the flattened configuration of the vessel defines an extended transverse diameter, a reduced lateral diameter, and a smaller cross-sectional area than the cross-sectional area of the vessel in a resting configuration.

3. The blood flow regulating device according to any of claims 1-2, wherein the vessel shaping device (100) is self-expanding to the expanded configuration.

4. The blood flow regulating device according to claim 3, wherein the vessel shaping device (100) is formed from Nitinol.

5. The blood flow regulating device according to any of claims 1-4, wherein the vessel shaping device (100) further comprises a tubular member (102) coupled to the frame (110).

6. The blood flow regulating device according to claim 5, wherein the tubular member (102) is fixedly attached to a distal collar (132) and slidably engaged with the proximal collar (130), the distal collar (132) defining an atraumatic tip.

7. The blood flow regulating device according to claim 5, wherein the tubular member (102) is slidably engaged with the distal collar (132) and fixedly attached to the proximal collar (130), a distal end of the tubular member (102) defining an atraumatic tip.

8. The blood flow regulating device according to any of claims 1-4, wherein movement of the tubular member (102) or an actuator rod (108) in one of a proximal direction or a distal direction further expands the vessel shaping device (100) along the transverse axis.

9. The blood flow regulating device according to claim 1, wherein the first stability member (116) or the second stability member (126) extends transversely inward towards the central longitudinal axis.

## Patentansprüche

1. Blutflussregulierungsvorrichtung für ein Gefäß, umfassend:
einen Zuführkatheter (90), der sich entlang einer Längsachse erstreckt; und
eine Gefäßformungsvorrichtung (100) mit einer zurückgezogenen Konfiguration in dem Zuführkatheter (90) und einer expandierten Konfiguration aus dem Zuführkatheter (90) heraus, wobei die Gefäßformungsvorrichtung (100) angeordnet ist, um sich entlang einer Querachse, die senkrecht zur Längsachse des Zuführkatheters (90) verläuft, in die expandierte Konfiguration auszudehnen, um das Gefäß in eine abgeflachte Konfiguration umzuformen, wobei die Gefäßformungsvorrichtung (100) einen Rahmen (110) umfasst einschließlich eines ersten Arms (112), der sich von der zentralen Längsachse aus quer nach außen erstreckt, um einen ersten Scheitelpunkt (114) zu definieren, und eines zweiten Arms (122), der sich von der zentralen Längsachse aus in einer dem ersten Arm (112) entgegengesetzten Richtung quer nach außen erstreckt, um einen zweiten Scheitelpunkt (124) zu definieren, wobei ein proximales Ende des ersten Arms (112) und ein proximales Ende des zweiten Arms (122) mit einem proximalen Kragen (130) verbunden sind und ein distales Ende des ersten Arms (112) und ein distales Ende des zweiten Arms (122) mit einem distalen Kragen (132) verbunden sind, **dadurch gekennzeichnet , dass**
die Gefäßformungsvorrichtung (100) weiter ein erstes Stabilitätselement (116), das sich über den ersten Scheitelpunkt von einem proximalen Abschnitt des ersten Arms (112) zu einem distalen Abschnitt des ersten Arms (112) erstreckt, und ein zweites Stabilitätselement (126), das sich über den zweiten Scheitelpunkt von einem proximalen Abschnitt des zweiten Arms (122) zu einem distalen Abschnitt des zweiten Arms (122) erstreckt, umfasst.

2. Blutflussregulierungsvorrichtung nach Anspruch 1, wobei die abgeflachte Konfiguration des Gefäßes einen erweiterten transversalen Durchmesser, einen reduzierten lateralen Durchmesser und eine kleinere Querschnittsfläche als die Querschnittsfläche des Gefäßes in einer Ruhekonfiguration definiert.

3. Blutflussregulierungsvorrichtung nach einem der Ansprüche 1-2, wobei die Gefäßformungsvorrichtung (100) sich selbst in die erweiterte Konfiguration ausdehnend ist.

4. Blutflussregulierungsvorrichtung nach Anspruch 3, wobei die Gefäßformungsvorrichtung (100) aus Nitinol besteht.

5. Blutflussregulierungsvorrichtung nach einem der Ansprüche 1-4, wobei die Gefäßformungsvorrichtung (100) weiter ein röhrenförmiges Element (102) umfasst, das mit dem Rahmen (110) gekoppelt ist.

6. Blutflussregulierungsvorrichtung nach Anspruch 5, wobei das röhrenförmige Element (102) fest an einem distalen Kragen (132) angebracht ist und verschiebbar mit dem proximalen Kragen (130) in Eingriff steht, wobei der distale Kragen (132) eine atraumatische Spitze definiert.

7. Blutflussregulierungsvorrichtung nach Anspruch 5, wobei das röhrenförmige Element (102) verschiebbar mit dem distalen Kragen (132) in Eingriff steht und fest mit dem proximalen Kragen (130) verbunden ist, wobei ein distales Ende des röhrenförmigen Elements (102) eine atraumatische Spitze definiert.

8. Blutflussregulierungsvorrichtung nach einem der Ansprüche 1-4, wobei die Bewegung des röhrenförmigen Elements (102) oder einer Betätigungsstange (108) in proximaler oder distaler Richtung die Gefäßformungsvorrichtung (100) entlang der Querachse weiter ausdehnt.

9. Blutflussregulierungsvorrichtung nach Anspruch 1, wobei sich das erste Stabilitätselement (116) oder das zweite Stabilitätselement (126) quer nach innen zur zentralen Längsachse erstreckt.

## Revendications

1. Dispositif de régulation d'écoulement sanguin pour un vaisseau, comprenant :
un cathéter d'administration (90) s'étendant le long d'un axe longitudinal ; et
un dispositif de mise en forme de vaisseau (100) ayant une configuration rétractée dans le cathéter d'administration (90) et une configuration déployée hors du cathéter d'administration (90), le dispositif de mise en forme de vaisseau (100) étant agencé pour se déployer dans la configuration déployée le long d'un axe transversal, perpendiculaire à l'axe longitudinal du cathéter d'administration (90), pour remettre en forme le vaisseau dans une configuration aplatie, dans lequel le dispositif de mise en forme de vaisseau (100) comprend un cadre (110) incluant un premier bras (112) s'étendant transversalement vers l'extérieur à partir de l'axe longitudinal central pour définir un premier sommet (114), et un second bras (122) s'étendant transversalement vers l'extérieur à partir de l'axe longitudinal central dans une direction opposée au premier bras (112) pour définir un second sommet (124), une extrémité proximale du premier bras (112) et une extrémité proximale du second bras (122) sont accouplées à un collier proximal (130), et une extrémité distale du premier bras (112) et une extrémité distale du second bras (122) sont accouplées à un collier distal (132), **caractérisé en ce que** :
le dispositif de mise en forme de vaisseau (100) comprend en outre un premier élément de stabilité (116) s'étendant à travers le premier sommet d'une partie proximale du premier bras (112) à une partie distale du premier bras (112), et un second élément de stabilité (126) s'étendant à travers le second sommet d'une partie proximale du second bras (122) à une partie distale du second bras (122).

2. Dispositif de régulation d'écoulement sanguin selon la revendication 1, dans lequel la configuration aplatie du vaisseau définit un diamètre transversal étendu, un diamètre latéral réduit et une zone de coupe transversale plus petite que la zone de coupe transversale du vaisseau dans une configuration de repos.

3. Dispositif de régulation d'écoulement sanguin selon l'une quelconque des revendications 1 à 2, dans lequel le dispositif de mise en forme de vaisseau (100) se déploie automatiquement vers la configuration déployée.

4. Dispositif de régulation d'écoulement sanguin selon la revendication 3, dans lequel le dispositif de mise en forme de vaisseau (100) est formé à partir de Nitinol.

5. Dispositif de régulation d'écoulement sanguin selon l'une quelconque des revendications 1 à 4, dans lequel le dispositif de mise en forme de vaisseau (100) comprend en outre un élément tubulaire (102) accouplé au cadre (110).

6. Dispositif de régulation d'écoulement sanguin selon la revendication 5, dans lequel l'élément tubulaire (102) est attaché de manière fixe à un collier distal (132) et mis en prise de manière coulissante avec le collier proximal (130), le collier distal (132) définissant un embout atraumatique.

7. Dispositif de régulation d'écoulement sanguin selon la revendication 5, dans lequel l'élément tubulaire (102) est mis en prise de manière coulissante avec le collier distal (132) et attaché de manière fixe au collier proximal (130), une extrémité distale de l'élément tubulaire (102) définissant un embout atraumatique.

8. Dispositif de régulation d'écoulement sanguin selon l'une quelconque des revendications 1 à 4, dans lequel le mouvement de l'élément tubulaire (102) ou d'une tige d'actionneur (108) dans une direction proximale ou une direction distale déploie davantage le dispositif de mise en forme de vaisseau (100) le long de l'axe transversal.

9. Dispositif de régulation d'écoulement sanguin selon la revendication 1, dans lequel le premier élément de stabilité (116) ou le second élément de stabilité (126) s'étend transversalement vers l'intérieur en direction de l'axe longitudinal central.
